# NEW EUROPEAN PATENT SPECIFICATION

(11) **EP 0 139 994 B2**
(45) Date of publication and mention of the opposition decision: **29.11.1995**
(45) Mention of the grant of the patent: 03.05.1989
(21) Application number: 84110126.4
(22) Date of filing: 24.08.1984
(51) Int. Cl.: A61L 2/18, A01N 59/00, G02C 13/00

(54) **Process and kit for disinfection of contact lenses**
Verfahren und "Kit" zur Desinfektion von Kontaktlinsen
Procédé et "kit" pour la désinfection de lentilles de contact

(30) Priority: 25.08.1983 US 526568
(43) Date of publication of application: 08.05.1985
(73) Proprietor: ALLERGAN, INC., Irvine, California 92713-9534 (US)
(72) Inventor: Kasper, Hans H., Saratoga, Calif. (US); Sibley, Murray J., Berkeley, Calif. (US); Yung, Gordon H., Santa-Clara, Calif. (US)
(74) Representative: Brauns, Hans-Adolf, Dr. rer. nat.

(56) References cited:
- EP-A- 124 461
- EP-A- 0 053 000
- EP-A- 0 082 798
- FR-A- 2 247 327
- FR-A- 2 256 767
- FR-A- 2 269 968
- FR-A- 2 282 987
- GB-A- 1 601 430
- GB-A- 2 003 033
- GB-A- 2 072 371
- JP-A- 5 241 209
- US-A- 3 623 492
- US-A- 3 873 696
- US-A- 4 180 467
- Pschyrembel W. "Klinisches Wörterbuch", page 875 ISBN 3-11-007018-9
- Hackh's Chemical Dictionary, 3rd edition, page 784
- A Gasset et al., "Hydrogen Peroxide Sterilisation of Hydrophilic Contact Lenses in Arch. Ophtalmol. Vol.93, June 1975, pages 412-415
- Physician's Desk Reference for Ophtalmology, 1985, page XIII
- Advertisement for Barnes-Hind Softmate Saline Spray

## Description

This application relates to the disinfection of contact lenses, and more particularly to disinfection by water-borne chemical agents without the application of heat.

Contact lenses accumulate dirt, proteinaceous matter, and microorganisms, all of which can adversely affect the health of the eye if allowed to accumulate on the lens. Therefore, the lenses must be cleaned and disinfected regularly and preferably daily.

It is generally known that hydrogen peroxide, in aqueous solution at a concentration of 3 wt.%, can be used to disinfect contact lenses and simultaneously remove unwanted dirt and proteinaceous matter. However, the hydrogen peroxide (hereafter, H₂O₂) will irritate the eye if even a small residual amount remains on the lens when it is re-inserted into the eye. This problem is especially notable with soft contact lenses, which are made from a water-permeable polymer into which the H₂O₂ can penetrate. Removing the H₂O₂ from such material is particularly difficult, and has required extensive washing and soaking with saline solution. Thus, it is desirable to be able to employ H₂O₂ in disinfecting contact lenses, while avoiding the irritancy of residual H₂O₂.

EP-A-82 798 discloses a process for disinfecting contact lenses comprising immersing the lenses in a container equipped with baskets and a solution of H₂O₂ at a concentration of 3 to 30%. At the end of the disinfection time (about 20 minutes), an agent for the decomposition of H₂O₂ within about 5 minutes (catalase) is added to said solution.

US-A-3 873 696 discloses a composition and a method of cleaning and sterilizing hydrophilic contact lenses with an aqueous, isotonic solution containing an effective amount of water soluble oxygen releasing salt such as potassium peroxymonosulfate.

U.S. Patent No. 3,829,329 discloses immersing sort contact lenses in a normal saline (NaCl) aqueous solution of 3% H₂O₂, shrink the lenses. The lenses were then boiled for 2 hours each in distilled water and normal saline solution. Adopting such a severe boiling regimen for the daily treatment of soft contact lenses would be inconvenient to the user and could cause the lenses to deteriorate.

U.S. Patent No. 3,908,680 discloses a treatment regimen for plastic articles such as contact lenses, which employs two boiling, aqueous baths containing a peroxy compound such as H₂O₂, followed by cleansing with a nonionic detergent and rinsing with distilled water. This sequence, besides being overly cumbersome for daily application, is not sure to remove all residual H₂O₂.

U.S. Patent No. 3,912,451 discloses that H₂O₂ in a solution used to sterilize soft contact lenses can be subsequently neutralized by contacting the solution with a metallic catalyst which decomposes the H₂O₂. This system is not sure to work quickly, as it relies on contact between the H₂O₂ and a solid metallic surface. Such a system also becomes less and less effective as the concentration of H₂O declines; unfortunately, the H₂O₂ concentration at which this system loses efficiency can still be high enough to risk irritation to the eye of the user. In addition, this system requires several manual or mechanical steps to bring the metallic catalyst into contact with the H₂O₂ solution. One such mechanical approach is disclosed in U.S. Patent No. 4,013,410, in which a mechanical timer rotates the container holding the solution so as to bring the solution into contact with a band of catalytic metal.

An article by A. R. Gasset et al., "Hydrogen Peroxide Sterilization of Hydrophilic Contact Lenses", in Arch. Opthalmol, Vol. 93 (June, 1975) pp. 412-415, discusses sterilizing sort contact lenses in 3% H₂O₂ solution. A solution of sodium thiosulfate was used to neutralize the residual H₂O₂ remaining in the lenses after sterilization. The authors found that sodium thiosulfate concentrations of 1.5% and 2.0% were unable to destroy all residual H₂O₂, while a 2.5% solution was effective. Adapting this technique to everday practice still requires the user to take several steps that are not all performed at the same time. This risks the omission of the neutralization step, through haste, carelessness, or forgetfulness, and also risks miscalculation of the amount and concentration of the sodium thiosulfate solution that is to be added.

The present invention provides a convenient, safe, process for disinfecting a contact lens. The process comprises:
(A) immersing the lens in a predetermined volume of an aqueous disinfecting solution of H₂O₂ at a concentration of 3 wt.% or less which is effective to disinfect the lens in a disinfection period of less than about 6 hours, and
(B) decomposing the H₂O₂ in the disinfecting solution by adding to the solution, before the disinfection period has elapsed at least the following additives:
   (1) one or more H₂O₂-neutralizing compounds which with H₂O₂ to form H₂O and reaction products which are non-injurious to the eye, the neutralizing compounds being present in an amount capable of reacting with all the H₂O₂ in the solution,
   (2) one or more water-soluble buffering agents in an amount from 0.1 to 1% per weight to provide the resulting reaction product containing aqueous solution with a pH of 6.5 to 8.5 and a tonicity of 200 to 450 milliosmol per kg of solution, characterized in that the additives being encased with a coating which dissolves gradually in the aqueous disinfecting solution, releasing one or more of the H₂O₂-neutralizing compounds and water-soluble agents after the disinfection period has elapsed to provide a buffered saline lens storage solution.

This process is particulary useful when the lens and the additives in the form of a tablet are placed in the H₂O₂ solution 5 minutes or less apart, and more preferably within 1 minute or less or simultaneously and when the H₂O₂ concentration is 3% or less.

The present invention effectively disinfects hard and soft contact lenses, especially soft lenses, i.e. lenses made of a hydrophilic polymer such as poly(hydroxyethylmethacrylate). Other such materials are well known to those skilled in this art. The invention can be carried out in an apparatus which holds the lenses immersed in an aqueous solution while permitting the solution to contact all surfaces of both lenses. One such device is known as the "Hydra-Mat® II", described in U.S. Patent No. 3,623,492. The present invention will be described with reference to that device. It includes two perforated baskets for holding a pair of contact lenses inside a container for a cleaning solution. The baskets are connected through gears to a knob on the cap of the container; twisting the knob spins the baskets in the cleaning solution.

The lens to be disinfected by the present invention is first removed from the eye and then cleaned, preferably by gentle rubbing between the fingers with a commercial lens cleaning solution for 15-20 seconds or by other appropriate methods for cleaning. This step, while not an essential part of the invention, helps loosen soil from the lens surface. The loosened soil and the cleaning solution are then rinsed off the lens with saline solution or tap water, or can be washed off by placing the lens into the Hydra-Mat® II baskets, adding saline solution to the container, and agitating the baskets by turning the top knob.

To disinfect the lens, it is immersed in an aqueous solution of H₂O₂ at a concentration of 3 wt.% or less than about 3 wt.%, e.g. as low as 1 wt.%, preferably as low as 0.25 wt.%, and even as low as 0.1 wt.%. The lens can be immersed in the H₂O₂ solution by placing it in one lens-holding basket of the Hydra-Mat® II device, and filling enough of the H₂O₂ solution into the container (after removing any other washing solutions) so that closing the cap over the container submerges the lens in the solution.

The low concentration of H₂O₂ that can be used in this process is noteworthy because the lens can be effectively disinfected in a reasonably short time. The low concentration also provides other advantages. One advantage in particular is that there is a reduced risk of harm to the eye from residual peroxide remaining in the lens. Another advantage is that lower peroxide concentrations cause little or no harm to the lens itself through chemical degradation of the polymeric structure. There should be a sufficient concentration of H₂O₂ that the lens is disinfected in a time up to about 6 hours; this corresponds to an H₂O₂ concentration of at least about 0.25 wt.%.

The concentration of H₂O₂ determines the length of time ("the disinfection period") for which the lens should be kept immersed in the H₂O₂ for disinfection. At H₂O₂ concentrations of 0.25 wt.%, the lens should be immersed in the H₂O₂ solution for about 6 hours to be sure of satisfactorily complete distinfection, whereas at 0.5 wt.% H₂O₂, the immersion time should be about 3 hours; and at 1.0 wt.% H₂O₂, immersion should be for about 2 hours, whereas a concentration of 3.0 wt.% H₂O₂ calls for immersion lasting about 5 minutes. One skilled in this art can readily determine the appropriate times for other initial concentrations of H₂O₂.

Another distinct and unobvious advantage of this process is the means by which the peroxide is removed from the lens. Specifically, a tablet is provided which is used to neutralize the H₂O₂ after a predetermined period of time and to transform the H₂O₂ solution into an essentially H₂O₂-free, buffered saline solution in which the lens can be safely stored. The lens can be removed immediately from the neutralized solution and reinserted into the eye, or it can be stored in the solution for a lengthy period of time, such as overnight.

In the broadest aspect of this invention, the tablet is placed into the H₂O₂ solution in which the lens is immersed at any time before the lens is completely disinfected, and the components of the tablet are released into the H₂O₂ solution only after the lens has been in the solution long enough to be disinfected. In the preferred embodiment of the invention, the lens and the tablet are placed into the H₂O₂ solution at about the same time, that is, within 5 minutes of each other, preferably within one minute, and more preferably essentially simultaneously.

The present invention, in its preferred embodiment in which the tablet and lens are placed into the H₂O₂ solution at essentially the same time, is particularly advantageous because it lets the lens wearer perform all the necessary steps at one time. By placing the tablet and lens into the H₂O₂ together, the user does not need to return later to remove residual H₂O₂ from the lens or to discard the H₂O₂ solution. Thus, there is no chance of the user forgetting to carry out such a subsequent step. In addition, once the lens, tablet, and H₂O₂ are sealed into a container (such as the Hydra-Mat® II) the user does not need to break sterility, i.e. he or she does not need to invade the sterilized environment of the lens-H₂O₂ system, and risk re-infecting the lens, to get rid of the H₂O₂ solution. The tablet destroys the H₂O₂ for the user, inside the sterilized environment.

The tablet includes one or more compounds that can react with H₂O₂ to form H₂O and byproducts that do not injure the lens or the eye of the lens wearer. The most preferred neutralizer is sodium thiosulfate. Neutralizers that can be used include thiourea (H₂NC(C)NH₂), sodium sulfite (Na₂SO₃), thioglycerol (HOCH₂CH(OH)CH₂SH), sodium formate (HCOONa), ascorbic acid 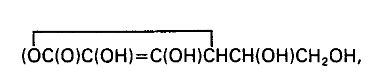
isoascorbic acid (erythorbic acid) 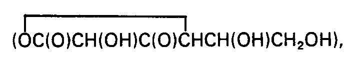
oxalic acid ((̵COOH)₂), glyoxylic acid (OCHCOOH), and tartaric acid ((̵CHOHCOOH)₂). Other neutralizers can work catalytically, such as platinum or other metals belonging to Period 4,5, or 6 of the Periodic Table, or peroxidase/catalase enzyme, but these are expensive, and do not cooperate to form a buffered lens storage solution.

The amount of the neutralizer to include in the tablet should be sufficient to react with all the H₂O₂ contained in the solution in which the lens is immersed. If sodium thiosulfate is employed, there should be at least a stoichiometric amount based on the amount of H₂O₂, that is, 1 mole of Na₂S₂O₃ per mole of H₂O₂. A small excess is acceptable but higher amounts of neutralizer are not necessary. The final concentrations of H₂O₂ in the solution after the neutralizer is dissolved and reaction has occurred should be less than 40 ppm, and is preferably less than 10 ppm. The neutralization is usually complete within 2 hours.

The tablet also contains water-soluble buffering agents to provide that when the tablet dissolves, and the H₂O₂ is completely neutralized, the resulting solution of buffering agents, neutralization reaction products, and excess neutralizer (if any) is a stable, buffered solution in which the lens can be safely stored. That is, the resultant solution should have a pH value of 6.5 to 8.5, and more preferably 7.0 to 7.9. The tonicity of the solution should be such that the osmotic pressure of the solution will permit the immersed lens to retain its normal shape, and thus should be about 200 to about 450 milliosmolar (mOs) per kg of solution. Those familiar with this art will recognize that this range corresponds to a range of about 0.6-1.4% NaCl equivalents. The solution should be isotonic with eye fluids, or up to about 1.4% hypertonic.

Satisfactory buffering agents include a mixture of sodium borate and boric acid in amounts which permit the above pH and tonicity to be attained. Typical amounts of a sobium borate/boric acid buffer are about 0.1 to about 1.0 wt.% of the eventual solution. Other buffering systems that can be used include systems based on monobasic and dibasic sodium phosphate; sodium citrate; or sodium carbonate. It will be recognized that the amount of buffering agent is a function of the initial liquid volume of the H₂O₂ solution, and of the amount of neutralizing agent (because its reaction products, e.g. Na⁺ and SO₃⁻² ions, will also influence the pH and tonicity). The amount of buffering agent can readuly be determined by those skilled in this art, with reference to the parameters mentioned herein.

The tablet optionally, and preferably, also contains a small amount (less than 1 wt.%) of a chelating agent such as EDTA to complex trace metals that may be present in the system. This additive helps retain the stability of the other agents in the solution, and helps any preservative that may be present retain its activity. Examples of suitable preservatives for inclusion in the tablet include potassium sorbate, sorbic acid, thimerosal, chlorhexidine, methyl and propyl parabens, chlorbutanol, benzalkonium chloride, and phenyl mercuric acetate and nitrate. These preservatives protect the tablet but more importantly act in solution, following dissolution of the tablet, to protect the lens. The preservative can comprise up to about 5.0 wt.% of the tablet.

The tablet can also include an acid and a base which form an effervescent couple that react in solution to generate a stream of CO₂ bubbles when the tablet components dissolve. The base should preferably be sodium bicarbonate, and the acid is preferably a weak organic acid such as citric or malic acid. The acid and base should be present in amounts which are approximately equimolar, and the acid and base together can comprise up to about 10 wt.% of the tablet. The CO₂ bubbles formed by reaction between the acid and base can aid the dispersion of the other tablet components in the H₂O₂ solution, and can thereby decrease the time needed to neutralize the solution. Alternatively, the user can agitate the container of solution (as by twisting the cap of the Hydra-Mat II) after the coating around the tablet has dissolved, to assist the dispersion of the tablet components.

The tablet (by which we include capsules and the like) has a coating which provides that the neutralizing and buffering agents in the tablet will come into contact with, and dissolve into, the H₂O₂ solution only a predetermined length of time after the tablet is placed into the solution. The coating is made of material which is dissolved or decomposed gradually by H₂O₂; suitable materials include organically modified cellulose, such as hydroxypropylmethyl cellulose, ethyl cellulose, cellulose acetate phthalate, hydroxypropyl cellulose; polyvinyl alcohol; and dibutyl phthalate. The thickness of the coating can readily be determined as follows. The length of time (t_{c}) for which the coating must keep the other agents from dissolving is readily determined from the length of the disinfection period (t_{d}), which as discussed above is a function of the concentration of the H₂O₂ solution that is being used. If the lens and the tablet are placed into the H₂O₂ solution at the same time, then t_{c} equals t_{d}. If the tablet is to be added a higher number of minutes after the lens is placed into the H₂O₂ solution, then t_{c} is less than t_{d} by that number; if the tablet is to be added a higher number of minutes before the lens is immersed, then t_{c} exceeds t_{d} by that number. One can independently determine the rate at which a given coating material is dissolved by a solution of H₂O₂ at the concentration being used, and by multiplying t_{c} by the rate of the H₂O₂ concentration being used, one can calculate how much of that coating to apply to a tablet for use with an H₂O₂ solution having that concentration. It is advantageous to make the coating 5 to 15% thicker than the calculated value, to ensure that the lens is exposed to unneutralized H₂O₂ for a sufficient disinfection time and to compensate for any slight loss of H₂O₂ due to reaction with the coating material.

The tablet can be made by dry blending the neutralizing and buffering agents, as well as the optional preservative and chelating agent, to obtain a uniform mixture, and then tabletting the blend employing conventional tabletting machinery. Separately, an amount of the coating material sufficient to form coatings of the desired thickness is dissolved in a solvent such as ethanol or acetone, and the solution is sprayed onto the tablets so as to form a uniform coating. The solvent is then evaporated, leaving the desired coating.

It will be recognized that all components of the tablet should be non-irritating to the eye when in solution. Standard tests for determining non-irritability to the eye are known to those skilled in this field. The H₂O₂ solutions can contain up to 0.05 wt.% of a stabilizer, and if so it should not react with or discolor the lens. A satisfactory stabilizer is a mixture of sodium stannate and sodium pyrophosphate, in a ratio of about 60:40 to 40:60 by weight.

The invention is described in the following Examples:

### Examples 1-6

Mixtures of materials were compounded into preparations having the composition given below. Each preparation was added to 7 ml of an aqueous solution of 0.5 wt.% H₂O₂. The time required for neutralization, and the pH and tonicity of the final solution, are also given. In each case, the final concentration of H₂O₂ was less than 40 ppm. Examples 2 and 6 were effervescent, i.e., on dissolution bubbles of CO₂ formed which dispersed the other components throughout the solution.

| Example No. | 1 | 2 | 3 | 4 | 5 | 6 |
|---|---|---|---|---|---|---|
| Ingredients (mg): | | | | | | |
| Sodium thiosulfate | 190 | 180 | 180 | 180 | 180 | 190 |
| Potassium sorbate | 10 | - | - | 10 | 10 | 10 |
| Disodium EDTA | 10 | 2 | - | 2 | 2 | 10 |
| Boric acid | 42 | - | 7.5 | 7.5 | 15 | 42 |
| Sodium bicarbonate | - | 12 | 5 | 5 | 10 | 12 |
| Citric acid | - | 8 | - | - | - | 8 |
| Sofium phosphate (monobasic) | - | - | 7 | 7 | - | - |
| Sodium phosphate (dibasic) | - | - | 10 | 10 | - | - |
| Time (hr) to complete neutralization | 1-2 | 4 | 4 | 4 | 3 | 1-2 |
| pH of neutralized solution | 7.9 | 7.3 | 7.2 | 7.0 | 7.9 | 7.9 |
| Tonicity (mOs/kg) of neutralized solution | 410. | 275. | 303. | 319. | 316. | 410. |

Another embodiment of the invention comprises a kit for disinfecting a contact lens in a predetermined volume of an aqueous disinfecting solution of H₂O₂ having a predetermined concentration, and for neutralizing the H₂O₂ in the solution after a disinfection period which is a function of the predetermined concentration and for transforming the solution *in situ* into a buffered isotonic lens storage solution; the kit comprising means for washing the lens, and a tablet, wherein said tablet comprises
(1) one or more H₂O₂-neutralizing compounds which react with H₂O₂ to form H₂O and reaction products which are non-injurious to the eye, the neutralising compounds being present in an amount effective to react with all the H₂O₂ in the disinfecting solution,
(2) one or more water-soluble buffering agents in an amount effective to provide the resulting reaction product containing aqueous solution with a pH of 6.5 to 8.5 and a tonicity of 200 to 450 milliosmol per kg of solution, and
(3) a coating encasing the tablet which coating when placed in the H₂O₂ solution before the end of the disinfection period, gradually dissolves in the H₂O₂ solution, and releases one or more neutralizing compounds and water-soluble agents only after said disinfection period has elapsed.

The means for washing the lens comprises a container open at its top for receiving said predetermined volume of said H₂O₂ solution and a lens case agitator;
a lidlike member removably mounted on the top end of the container and open at both ends and having a transverse partition intermediate its ends;
Said partition having an aperture extending therethrough;
a lens case agitator pivotally mounted in said aperture in the partition and extending into said container and having depending lens case supporting means and an upper end extending above the partition and having a spur gear thereon;
a knoblike member pivotally received in the upper end of the lidlike member and having finger grasp means for rotating the same and internal gear teeth therein;
planetary gear means pivotally mounted on said transverse top portion of the lidlike member and interposed between said spur gear on the agitator and said internal gear teeth in the knoblike member for imparting rotation to said agitator member whereby a lens carried in said lens case is washed in the liquid in the container.

In the kit the coating is characterized in that when the tablet is placed in said H₂O₂ solution up to 5 minutes after the beginning of the disinfection period, the coating dissolves only after the disinfection period has elapsed.

The kit is employed in the manner described herein, to clean and disinfect a contact lens. The tablet's composition is predetermined to be used with a specified volume of aqueous H₂O₂ solution having a specified concentration, and instructions are provided with the kit to inform the user of the strength and volume of the H₂O₂ solution to use. More advantageously, the kit includes a bottle of an aqueous solution of H₂O₂, whose concentration is predetermined (0.25-3 wt.%) in conjunction with the composition of the tablet. In this embodiment, the only measurement required of the user is pouring a specified amount of the solution into the container of the lens washer. The amount can be marked by a line provided on the container at the height which corresponds to the specified volume. The user is simply instructed to pour solution from the bottle to this line, to place the lenses and a tablet into the container, and to close the lid of the container.

## Claims

1. A process for disinfecting a contact lens, comprising:
(A) immersing the lens in a predetermined volume of an aqueous disinfecting solution of H₂O₂ at a concentration of 3 wt.% or less which is effective to disinfect the lens in a disinfection period of less than about 6 hours, and
(B) decomposing the H₂O₂ in the disinfecting solution by adding to the solution, before the disinfection period has elapsed at least the following additives:
(1) one or more H₂O₂-neutralizing compounds which react with H₂O₂ to form H₂O and reaction products which are non-injurious to the eye, the neutralizing compounds being present in an amount capable of reacting with all the H₂O₂ in the solution,
(2) one or more water-soluble buffering agents in an amount from 0.1 to 1% per weight to provide the resulting reaction product containing aqueous solution with a pH of 6.5 to 8.5 and a tonicity of 200 to 450 milliosmol per kg of solution, characterized in that the additives being encased with a coating which dissolves gradually in the aqueous disinfecting solution, releasing one or more of the H₂O₂-neutralizing compounds and water-soluble agents after the disinfection period has elapsed to provide a buffered saline lens storage solution.

2. The process of Claim 1 wherein the additives are in the form of a tablet.

3. The process of Claim 1 or 2 wherein the water-soluble agents are present in an amount effective to provide the resulting solution with a pH of about 7.0 to about 7.9.

4. The process of any of Claims 1 to 3 wherein the coating is an organically modified cellulose, polyvinyl alcohol, or dibutyl phthalate.

5. The process of any of Claims 2 to 4 wherein the lens and the tablet are placed into the disinfecting solution within 5 minutes of each other.

6. The process of Claim 5 wherein the lens and the tablet are placed into the solution within 1 minute of each other or simultaneously.

7. The process of any of Claims 1 to 6 wherein the additives may also contain up to about 10 wt.% of a mixture of an acid and a base which react in solution to form CO₂ gas, up to about 1% of a chelating agent and/or up to about 5 wt.% of a preservative.

8. The process of any of Claims 1 to 7 wherein the concentration of H₂O₂ in the buffered, saline lens storage solution is less than about 40 ppm.

9. The process of any of Claims 1 to 8 wherein the one or more H₂O₂-neutralizing compounds comprises sodium thiosulfate.

10. The process of any of Claims 1 to 9 wherein the water-soluble agents are a mixture of sodium borate and boric acid.

11. The process of any of Claims 1 to 10 wherein the disinfection period is less than 3 hours.

12. The process of any of Claims 1 to 10 wherein the concentration of the H₂O₂ is 1 wt.% or less.

13. A kit for disinfecting a contact lens in a predetermined volume of an aqueous disinfecting solution of H₂O₂ having a predetermined concentration, and for neutralizing the H₂O₂ in the solution after a disinfection period which is a function of the predetermined concentration and for transforming the solution *in situ* into a buffered isotonic lens storage solution; the kit comprising means for washing the lens, and a tablet wherein said tablet comprises
(1) one or more H₂O₂-neutralizing compounds which react with H₂O₂ to form H₂O and reaction products which are non-injurious to the eye, the neutralizing compounds being present in an amount effective to react with all the H₂O₂ in the disinfecting solution,
(2) one or more water-soluble buffering agents in an amount effective to provide the resulting reaction product containing aqueous solution with a pH of 6.5 to 8.5 and a tonicity of 200 to 450 milliosmol per kg of solution, and
(3) a coating encasing the tablet which coating when placed in the H₂O₂ solution before the end of the disinfection period, gradually dissolves in the H₂O₂ solution, and releases one or more neutralizing compounds and water-soluble agents only after said disinfection period has elapsed.

14. The kit of Claim 13 wherein said means for washing the lens comprises a container open at its top for receiving said predetermined volume of said H₂O₂ solution and a lens case agitator;
a lidlike member removably mounted on the top end of the container and open at both ends and having a transverse partition intermediate its ends;
said partition having an aperture extending therethrough;
a lens case agitator pivotally mounted in said aperture in the partition and extending into said container and having depending lens case supporting means and an upper end extending above the partition and having a spur gear thereon;
a knoblike member pivotally received in the upper end of the lidlike member and having finger grasp means for rotating the same and internal gear teeth therein;
planetary gear means pivotally mounted on said transverse top portion of the lidlike member and interposed between said spur gear on the agitator and said internal gear teeth in the knoblike member for imparting rotation to said agitator member whereby a lens carried in said lens case is washed in the liquid in the container.

15. The kit of Claim 14 or 15 wherein the coating is characterized in that when the tablet is placed in said H₂O₂ solution up to 5 minutes after the beginning of the disinfection period, the coating dissolves only after the disinfection period has elapsed.

16. The kit according to any of Claims 13 to 15 further comprising a bottle of an aqueous solution of H₂O₂ having said predetermined concentration.

## Patentansprüche

1. Verfahren zum Desinfizieren einer Kontaktlinse, umfassend:
(A) das Eintauchen der Linse in ein vorbestimmtes Volumen einer wässrigen Desinfizierungslösung aus H₂O₂ in einer Konzentration von 3 Gew.% oder weniger, welche die Linse in einem Desinfektionszeitraum von weniger als etwa 6 Stunden wirksam desinfizieren kann; und
(B) Zersetzen des H₂O₂ in der Desinfektionslösung durch Zugabe zu der Lösung, bevor der Desinfektionszeitraum abgelaufen ist, wenigstens der folgenden Additive:
(1) ein oder mehrere H₂O₂ neutralisierende Verbindungen, die mit H₂O₂ reagieren, unter Ausbildung von H₂O₂, und Reaktionsprodukten, die das Auge nicht schädigen, wobei die neutralisierenden Verbindungen in einer Menge vorliegen, die ausreicht, um mit dem gesamten, in der Lösung vorhandenen H₂O₂ zu reagieren,
(2) eine oder mehrere wasserlösliche Puffermittel in einer Menge von 0,1 bis 1 Gew.%, um die entstehende, das Reaktionsprodukt enthaltende, wässrige Lösung mit einem pH-Wert von 6,5 bis 8,5 zu versehen, und einer Tonizität von 200 bis 400 Milliosmol pro kg der Lösung; dadurch gekennzeichnet, dass die Additive in einer Beschichtung eingeschlossen sind, die sich allmählich in der wässrigen Desinfektionslösung auflöst und die ein oder mehrere H₂O₂ neutralisierende Verbindungen und wasserlösliche Mittel nach Ablauf der Desinfektionsperiode unter Ausbildung einer gepufferten Salz-Linsenlagerungslösung freigibt.

2. Verfahren gemäss Anspruch 1, bei dem die Additive in Form einer Tablette vorliegen.

3. Verfahren gemäss Anspruch 1 oder 2, bei dem die wasserlöslichen Mittel in einer Menge vorliegen, die ausreicht, um der erhaltenen Lösung einen pH-Wert von etwa 7,0 bis etwa 7,9 zu verleihen.

4. Verfahren gemäss einem der Ansprüche 1 bis 3, bei dem der Überzug eine organisch modifizierte Zellulose, Polyvinylalkohol oder Dibutylphthalat ist.

5. Verfahren gemäss einem der Ansprüche 2 bis 4, bei dem die Linse und die Tablette in die Desinfektionslösung in einem Abstand von 5 Minuten nacheinander eingelegt werden.

6. Verfahren gemäss Anspruch 5, bei dem die Linse und die Tablette in die Lösung in einem Abstand von 1 Minute oder gleichzeitig eingelegt werden.

7. Verfahren gemäss einem der Ansprüche 1 bis 6, bei dem die Additive ausserdem noch bis zu 10 Gew.% einer Mischung aus einer Säure und einer Base, welche in der Lösung unter Ausbildung von CO₂-Gas reagiert, bis zu etwa 1% eines Gelbildungsmittels und/oder bis zu etwa 5 Gew.% eines Konservierungsmittels enthält.

8. Verfahren gemäss einem der Ansprüche 1 bis 7, bei dem die Konzentration an H₂O₂ in der gepufferten Salz-Linsenaufbewahrungslösung weniger als etwa 40 ppm beträgt.

9. Verfahren gemäss einem der Ansprüche 1 bis 8, bei welchem die eine oder mehreren H₂O₂ neutralisierende Verbindung(en) Natriumthiosulfat umfasst.

10. Verfahren gemäss einem der Ansprüche 1 bis 9, bei dem die wasserlöslichen Mittel eine Mischung aus Natriumborat und Borsäure sind.

11. Verfahren gemäss einem der Ansprüche 1 bis 10, bei dem der Desinfektionszeitraum weniger als 3 Stunden beträgt.

12. Verfahren gemäss einem der Ansprüche 1 bis 10, bei dem die Konzentration des H₂O₂ 1 Gew.% oder weniger beträgt.

13. Gebinde zum Desinfizieren einer Kontaktlinse in einem vorbestimmten Volumen einer wässrigen Desinfektionslösung von H₂O₂ mit einer vorbestimmten Konzentration und zum Neutralisieren des H₂O₂ in der Lösung nach einem Desinfektionszeitraum, der eine Funktion der vorbestimmten Konzentration ist, und zum Überführen der Lösung in situ in eine gepufferte, isotonische Linsenlagerungslösung, wobei das Gebinde Einrichtungen zum Waschen der Linse und eine Tablette umfasst, und wobei die Tablette umfasst:
(1) eine oder mehrere H₂O₂ neutralisierende Verbindungen, die mit H₂O₂ unter Bildung von H₂O und für das Auge nicht schädlichen Reaktionsprodukten reagieren, wobei die neutralisierenden Verbindungen in einer Menge vorliegen, die wirksam ist, um mit dem gesamten H₂O₂ in der Desinfektionslösung zu reagieren,
(2) eine oder mehrere wasserlösliche Puffermittel in einer Menge, die wirksam ist, um in der das erhaltene Reaktionsprodukt enthaltenden wässrigen Lösung einen pH-Wert von 6,5 bis 8,5 und eine Tonizität von 200 bis 450 Milliosmol pro kg der Lösung zu ergeben, und
(3) eine Beschichtung, welche die Tablette einschliesst, wobei sich die Beschichtung, wenn man sie in die H₂O₂-Lösung vor Ende des Desinfektionszeitraums gibt, allmählich in der H₂O₂-Lösung auflöst und ein oder mehrere neutralisierende Verbindungen und wasserlösliche Mittel erst nach Ablauf des Desinfektionszeitraumes freigibt.

14. Gebinde nach Anspruch 13, worin die Vorrichtung zum Waschen der Linsen einen an seinem oberen Ende offenen Behälter zur Aufnahme des vorbestimmten Volumens der H₂O₂-Lösung und eine Linsenbehälter-Rührvorrichtung, ein
deckelartiges Element, das abnehmbar auf dem oberen Ende des Behälters angebracht und an beiden Enden offen ist, und ein Quertrennung zwischen seinen Enden aufweist,
die Trennung ein sich dadurch erstreckende Öffnung aufweist,
eine Linsenbehälter-Rührvorrichtung, die schwenkbar in der Öffnung in der Trennung abgebracht ist und sich in den Behälter erstrecktm und eine herunterhängende Linsenbehälter-Trägervorrichtung und ein oberhalb der Trennung sich erstreckendes oberes Ende sowie ein darauf angeordnetes Stirnradgetriebe aufweist,
ein knopfartiges Element, das schwenkbar in dem oberen Ende des deckelartigen Elementes aufgenommen ist und eine mit den Fingern zu greifende Vorrichtung zum Drehen derselben und eine innere Getriebeverzahnung darin aufweist,
eine Planetengetriebevorrichtung, die schwenkbar auf dem querliegenden, oberen Endabschnitt des deckelartigen Elementes angebracht und zwischen dem Stirnradgetriebe auf der Rohrvorrichtung und der inneren Getriebeverzahnung in das knopfartige Element eingesetzt ist, um der Rührvorrichtung ein Drehen zu verliehen, wobei eine in dem Linsenbehälter getragene Linse in der Flüssigkeit des Behälters gewaschen wird, umfasst.

15. Gebinde gemäss Ansprüchen 13 oder 14, bei dem der Überzug dadurch gekennzeichnet ist, dass dann, wenn man die Tablette in die H₂O₂-Lösung bis zu 5 Minuten nach Beginn des Desinfektionszeitraumes einbringt, sich der Überzug erst nach Ablaufen des Desinfektionszeitraumes auflöst.

16. Gebinde gemäss einem der Ansprüche 13 bis 15, enthaltend weiterhin eine Flasche mit einer wässrigen Lösung von H₂O₂, welches die vorbestimmte Konzentration aufweist.

## Revendications

1. Procédé de désinfection d'une lentille de contact, consistant à:
(A) immerger la lentille dans un volume prédéterminé d'une solution désinfectante aqueuse d'H₂O₂, à une concentration de 3% en poids, ou moins, qui est efficace pour désinfecter la lentille en une période de désinfection de moins d'environ 6 heures; et
(B) décomposer l'H₂O₂ de la solution désinfectante en ajoutant à la solution, avant que la période de désinfection ne soit écoulée, au moins les additifs suivants:
(1) un ou plusieurs composés neutralisant H₂O₂, qui réagissent avec H₂O₂ pour former H₂O et des produits de réaction qui ne sont pas dangereux pour l'oeil, les composés neutralisants étant présents en une quantité capable de réagir avec la totalité de l'H₂O₂ de la solution,
(2) un ou plusieurs agents tampons solubles dans l'eau, en une quantité allant de 0,1 à 1% en poids pour doter la solution aqueuse contenant le produit de réaction résultant d'un pH de 6,5 à 8,5 et d'une tonicité de 200 à 450 milliosmol par kg de solution, caractérisé par le fait que les additifs sont enfermés dans un enrobage qui se dissout progressivement dans la solution désinfectante aqueuse, libérant l'un ou plusieurs des composés neutralisant H₂O₂ et des agents solubles dans l'eau après que la période de désinfection se soit écoulée, pour fournir une solution saline tamponnée de stockage de la lentille.

2. Procédé selon la revendication 1, caractérisé en ce que les additifs se présentent sous la forme d'un comprimé.

3. Procédé selon l'une des revendications 1 ou 2, caractérisé en ce que les agents solubles dans l'eau sont présents en une quantité efficace pour doter la solution résultante d'un pH d'environ 7,0 à environ 7,9.

4. Procédé selon l'une des revendications 1 à 3, caractérisé en ce que l'enrobage est une cellulose modifiée organiquement, de l'alcool polyvinylique, ou du phtalate de dibutyle.

5. Procédé selon l'une des revendications 2 à 4, caractérisé en ce que l'on place la lentille et le comprimé dans la solution désinfectante à 5 minutes ou moins d'intervalle.

6. Procédé selon la revendication 5, caractérisé en ce que l'on place la lentille et le comprimé dans la solution à 1 minute ou moins d'intervalle ou simultanément.

7. Procédé selon l'une des revendications 1 à 6, caractérisé en ce que les additifs peuvent également contenir jusqu'à environ 10% en poids d'un mélange d'un acide et d'une base qui réagissent en solution pour former CO₂ gazeux, jusqu'à environ 1% en poids d'un agent chélatant et/ou jusqu'à environ 5% en poids d'un agent conservateur.

8. Procédé selon l'une des revendications 1 à 7, caractérisé en ce que la concentration d'H₂O₂ dans la solution saline tamponnée de stockage de la lentille est inférieure à environ 40 ppm.

9. Procédé selon l'une des revendications 1 à 8, caractérisé en ce que un ou plusieurs composés neutralisant H₂O₂ cmprennent du thiosulfate de sodium.

10. Procédé selon l'une des revendications 1 à 9, caractérisé en ce que les agents solubles dans l'eau sont un mélange de borate de sodium et d'acide borique.

11. Procédé selon l'une des revendications 1 à 10, caractérisé en ce que la période de désinfection est inférieure à 3 heures.

12. Procédé selon l'une des revendications 1 à 10, caractérisé en ce que le concentration de l'H₂O₂ est de 1% en poids ou moins.

13. Nécessaire pour désinfecter une lentille de contact dans un volume prédéterminé d'une solution désinfectante aqueuse d'H₂O₂ ayant une concentration prédéterminée, et pour neutraliser l'H₂O₂ de la solution après une période de désinfection, qui est fonction de la concentration prédéterminée et pour transformer la solution *in situ* en une solution isotonique tamponée de stockage de la lentille, le nécessaire étant caractérisé en ce qu'il comprend des moyens pour laver la lentille, et un comprimé, ledit comprimé comprenant:
(1) un ou plusieurs composés neutralisant H₂O₂, qui réagissent avec H₂O₂ pour former H₂O et des produits de réaction qui ne sont pas dangereux pour l'oeil, les composés neutralisants étant présents en une quantité efficace pour réagir avec la totalité de l'H₂O₂ de la solution désinfectante;
(2) un ou plusieurs agents tampons solubles dans l'eau, en une quantité efficace pour doter la solution aqueuse contenant le produit de réaction résultant d'un pH de 6,5 à 8,5 et d'une tonicité de 200 à 450 milliosmol par kg de solution; et
(3) un enrobage enfermant le comprimé, ledit enrobage, une fois placé dans la solution d'H₂O₂ avant la fin de la période de désinfection, se dissolvant progressivement dans la solution d'H₂O₂, et libérant un ou plusieurs composés neutralisants et agents solubles dans l'eau seulement après que ladite période de désinfection de soit écoulée.

14. Nécessaire selon la revendication 13, caractérisé en ce que lesdits moyens pour laver la lentille comprennent un récipient ouvert à sa partie supérieure pour recevoir ledit volume prédéterminé de ladite solution d'H₂O₂ et un agitateur du boîtier des lentilles;
un élément en forme de couvercle, monté de façon amovible sur l'extrémité supérieure du récipient et ouvert aux deux extrémités et présentant une cloison transversale se trouvant entre ses extrémités, ladite cloison étant traversée par une ouverture;
un agitateur du boîtier des lentilles monté de façon pivotante dans ladite ouverture de la cloison et s'étendant dans ledit récipient et présentant des moyens supportant le boîtier des lentilles des façon suspendue et une extrémité supérieure s'étendant au-dessus de la cloison et sur laquelle est monté un engrenage droit;
un élément de type bouton reçu de façon pivotante dans l'extrémité supérieure de l'élément de type couvercle et présentant des moyens de saisie par les doigts pour faire tourner ledit élément et renfermant une denture d'engrenage interne;
des moyens d'engrenage planétaire montés de façon pivotante sur ledite partie supérieure transversale de l'élément de type couvercle et interposés entre ledit engrenage droit sur l'agitateur et ladite denture d'engrenage interne de l'élément de type bouton, pour faire tourner ledit élément d'agitateur, ce par quoi une lentille supportée dans ledit boîtier des lentilles est lavée dans le liquide du récipient.

15. Nécessaire selon l'une des revendications 14 ou 15, caractérisé en ce que le revêtement est caractérisé par le fait que, lorsque le comprimé est placé dans ladite solution d'H₂O₂ pendant un temps allant jusqu'à 5 minutes après le début de la période de désinfection, l'enrobage ne se dissout qu'après que la période de désinfection se soit écoulée.

16. Nécessaire selon l'une des revendications 13 à 15, caractérisé en ce qu'il comprend en outre un flacon d'une solution aqueuse d'H₂O₂ ayant ladite concentration prédéterminée.
